# EUROPEAN PATENT SPECIFICATION

(11) **EP 2 338 355 B1**
(45) Date of publication and mention of the grant of the patent: **27.04.2016**
(21) Application number: 10015331.1
(22) Date of filing: 06.12.2010
(51) Int. Cl.: C09B 61/00, C12P 23/00

(54) **Method for extracting carotenoids from plant matrices**
Verfahren zur Gewinnung von Karotenoiden aus pflanzlichem Material
Méthode pour l'extraction des carotenoïdes du matérial végétal

(30) Priority: 22.12.2009 IT AN20090102
(43) Date of publication of application: 29.06.2011
(73) Proprietor: Biosphere S.p.A., 47023 Cesena (Forli') (IT)
(72) Inventor: Argnani, Miriam, 47032 Bertinoro (FC) (IT); Lavecchia, Roberto, Roma (IT); Pistocchi, Marco, 47032 Bertinoro (FC) (IT); Zuorro, Antonio, Roma (IT)
(74) Representative: Cacciamani, Clizia

(56) References cited:
- US-A- 5 830 738
- ROBERTO LAVECCHIA ET AL: "Improved lycopene extraction from tomato peels using cell-wall degrading enzymes", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 228, no. 1, 10 June 2008 (2008-06-10) , pages 153-158, XP019653032, ISSN: 1438-2385, DOI: DOI:10.1007/S00217-008-0897-8
- SANTAMARIA R I ET AL: "Selective enzyme-mediated extraction of capsaicinoids and carotenoids from chili guajillo puya (Capsicum annuum L.) using ethanol as solvent", JOURNAL OF AGRICULTURAL AND FOOD CHEMISTRY, AMERICAN CHEMICAL SOCIETY, US, vol. 48, no. 7, 1 July 2000 (2000-07-01), pages 3063-3067, XP002314984, ISSN: 0021-8561, DOI: DOI:10.1021/JF991242P
- CHOUDHARI ET AL: "Enzyme aided extraction of lycopene from tomato tissues", FOOD CHEMISTRY, ELSEVIER LTD, NL, vol. 102, no. 1, 5 December 2006 (2006-12-05), pages 77-81, XP005793734, ISSN: 0308-8146, DOI: DOI:10.1016/J.FOODCHEM.2006.04.031
- ADVISORY COMMITTEE FOR NOVEL FOODS AND PROCESSES: 'Tomato oleoresin containing lycopene for use in foods for special medical purposes (lycored)', [Online] 01 August 2008, Retrieved from the Internet: <URL:http://www.food.gov.uk/multimedia/pdfs /acnfp9005> [retrieved on 2013-02-05]
- EFSA EUROPEAN FOOD SAFETY AUTHORITY: 'Safety of Lycopene oleoresin from tomatoes', [Online] 30 April 2008, Retrieved from the Internet: <URL:http://www.efsa.europa.eu/de/efsajourn al/doc/675.pdf> [retrieved on 2013-02-05]

## Description

The present invention relates to the recovery and purification of naturally occurring substances from their matrix, and particularly it relates to a method for extracting carotenoid substances from a plant-derived matrix.

Carotenoids are pigments contained in plants and other photosynthetic organisms such as algae, where they have an important role in the process of photosynthesis. For example, lycopene is mainly contained in tomatoes, beta-carotene is contained in carrots, and lutein is contained in green-leafy vegetables such as spinaches.

Carotenoids are chemically divided into carotenes whose molecules are formed only by carbon and hydrogen atoms (for example, beta-carotene and lycopene), and xanthophylls whose molecules also contain oxygen atoms (for example, lutein).

For a long time the attention of chemical industry, food industry and recently also pharmaceutical industry has been directed toward carotenoid substances due to the plenty of properties of these molecules. In fact, carotenoids are valuable natural dyes for food industry (for example, lycopene is a dye known as E160d and lutein as E161 b), however they also have notable antioxidant properties; furthermore, several clinical studies showed that certain carotenoids can prevent tumour diseases.

The increasing interest toward this type of molecules and, from a biorefinery perspective, the ability to combine and integrate the extraction process of carotenoids with an existing agri-food process led to the research and development of the present invention.

Carotenoids can be industrially obtained by extraction from fruit and vegetables containing them in considerable amounts with organic solvents or supercritical fluids, or by fermentation of certain species of microorganisms (fungi or bacteria), or by chemical synthesis.

To date, several problems related to the recovery and purification of carotenoids from plant products have been addressed. Generally, the core problem is to be able to recover such organic molecules from a matrix which is substantially cellulosic in nature and most frequently encountered in a predominantly aqueous environment. Furthermore, the desired process should be robust and efficient so that it can be applied to various plant matrixes: by way of a non-exhaustive example, fresh fruit and vegetables, products and by-products of preserve-processing industry (juices, pulps, concentrates, etc.), and even their waste products. To this aim, methods have been developed to extract these molecules comprising the use of a particular mixture of enzymes which can assist in the break-down of the cellulose matrix so as to make carotenoids more susceptible to be extracted with a solvent.

WO03/020053 discloses a process for extracting carotenoids from tomatoes, carrots, wolfberries; plant material is mixed with water to achieve a Brix value not greater than 10, thereafter the plant material is crushed and pulp is separated from serum by employing conventional decanting, centrifugation or filtration techniques; the pulp is then extracted with a solvent and the oleoresin is recovered by evaporation of the solvent.

WO97/48287 discloses the separation of pulp from serum by means of a decanter. The pulp is subjected to extraction.

US5830738 discloses a process for extracting carotenoids from plant material in which it is stated that plant material must be crushed because the smaller is the size of the material, the larger is the contact surface with the enzyme. Plant matrix is treated with enzymes to assist in breaking down plant cell walls and then, after being separated from the aqueous phase containing the enzymes, it is extracted with solvent to recover carotenoids.

Food Chemistry 102 (2007) 77-81 discloses tests on extraction of lycopene from tomatoes (peel, pulp and waste from tomato processing industry) after an enzymatic treatment with cellulase and pectinase enzymes in various ratios. The enzyme-treated plant matrix is filtered and the residual solid is repeatedly extracted with solvents such as petroleum ether and acetone.

J. Agric. Food Chem. 2000, 48, 3063-3067 discloses the enzymatic pre-treatment of a plant matrix of chili fruits with cellulase and pectinase enzymes to increase the extraction yield of capsaicinoids and carotenoids. Plant residue is dried and then extracted with solvent.

The last three documents reported above disclose processes which all include an intermediate step of separation between the enzymatic treatment and the extraction with solvent, using conventional separation techniques as centrifugation or filtration; the cost of this separation step is significant with respect to the investment for carrying out the process on an industrial scale and, moreover, it is a potential source of product loss.

Patent Application RM2007A000540 discloses a method which includes extracting carotenoids from a plant matrix by means of two immiscible phases; which two immiscible phases include an aqueous phase containing one or more enzymes, and a non-aqueous phase consisting of the solvent which is non-polar or poorly polar. This procedure has the advantage of contacting the aqueous phase containing the enzymes with a non-polar phase in which carotenoids are much more soluble, so as to eliminate an intermediate step of separation. The problem of this method is mainly that it has been developed in a laboratory environment and there are not reported any extensive studies about the issues related to its use on an operational scale, neither at the level of a pilot plant nor at the level of a true industrial plant.

Eur. Food. Res. Technol., 228: 153-158 2008 discloses the extraction of lycopene from tomato peels by means of a pre-treatment with enzymes (cellulases, hemicellulases and pectinases) and a subsequent extraction with solvent. The article shows that the use of enzymes improves the extraction yield, however there are reported only examples which make use of each enzyme separately - not in mixture - and only on a laboratory scale.

Therefore, an aim of the present invention is to provide an improved method for extracting carotenoid substances from a plant matrix which can be applied to industrial processes in order to obtain significant results, both in terms of purity of the product and in terms of economy and efficiency of the process itself.

Accordingly, the object of the present invention is a method for extracting carotenoid substances from plant matrices, comprising the steps of: conditioning a plant matrix; treating the plant matrix with an enzymatic formulation in an aqueous phase; treating the mixture comprising the plant matrix and the enzymatic formulation with an organic solvent; separating the three phases, i.e. the aqueous phase containing the enzymatic formulation which is recycled, the organic phase containing carotenoids, and the solid phase, i.e. the plant matrix; evaporating the organic solvent and recovering an oleoresin; purifying the carotenoid substances contained in the oleoresin, wherein the enzymatic formulation includes pectinase, hemicellulase and cellulase enzymes having 1/6 pectinase activity, 1/6 hemicellulase activity and 4/6 cellulase activity..

In a preferred embodiment, the step of conditioning the plant matrix is carried out by grinding, so as to make the surface of said matrix more susceptible to processes which will be carried out thereon. In some cases, grinding can be followed by a step in which the plant matrix already finely ground is homogenized under a high pressure in order to further reduce the matrix in size. Lycopene, and particularly lycopene obtainable from tomato, is the carotenoid which was most investigated in the research that led to the present invention.

The enzymatic formulation includes such a plurality of enzymes to treat the plant matrix in a manner that can break the plant cell wall and thereby make carotenoid substances more available for the subsequent extraction with solvent. By way of a non-exhaustive example, commercially available enzymatic formulations having pectinase, hemi-cellulase and cellulase activities can be used, for example Pectinex, Viscozyme and Citrozym by Novozymes, or Peclyve, Brewlyve and Cellulyve by Lyven.

Preferably, the solvent is a non-polar aprotic solvent, particularly hexane or ethyl acetate or mixtures thereof.

Further advantages and features of the method according to the present invention will be apparent from the following detailed description of an embodiment thereof, which is provided by way of illustration, and not by way of limitation, with reference to the accompanying drawing wherein:

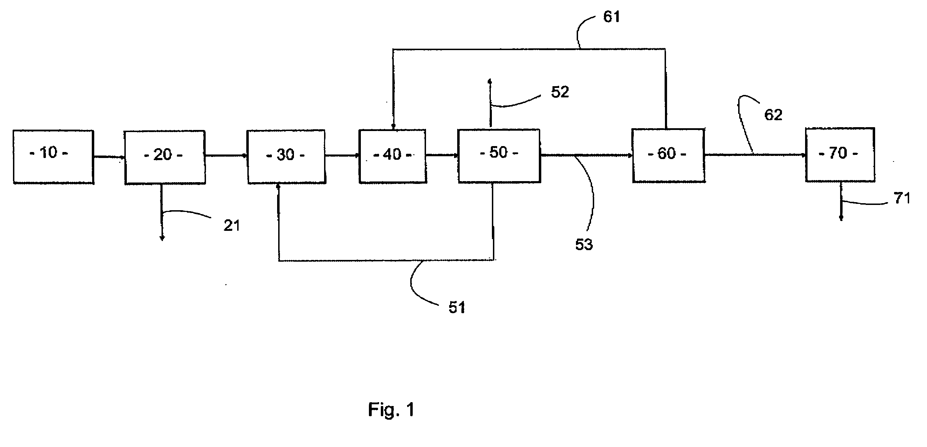

Figure 1 is a block diagram showing the steps of the method according to the present invention.

Figure 1 shows a block diagram of the method according to the present invention; reference numeral 10 denotes a step in which plant matrix is ground, followed by a centrifugation step 20 in which water is removed (21). Thereafter, following an enzymatic pre-treatment step 30, plant matrix is extracted 40 with an organic solvent; in the subsequent phase-separation step 50, the various phases are separated: the aqueous phase 51, the organic solvent 53 in which the carotenoid is contained, and the exhausted plant matrix 52. The aqueous phase 51 is then recycled into the pre-treatment step 30, as it contains enzymes which can be re-utilized in that step; the exhausted plant matrix 52 is sent to de-solventization; the organic phase 53 is subjected to evaporation 60 in order to recover neat solvent 61 which can be re-utilized in the extraction step 40, while the oleoresin 62 is subjected to purification 70 in order to obtain carotenoid crystals 71.

The operation of the method according to the present invention will be clear from the following. The grinding step is carried out by conventional known techniques, for example blade-, bead- or hammer-mills; preferably, a particular refiner is used which is provided with a series of vanes helically arranged on a shaft to grind the product and push it towards a sieve which allows ground pulp and peels to pass therethrough, while seeds and coarse matter are prevented to leave the apparatus; the mash obtained by the refiner is sent to the present process. The holes of the sieve are less than 3 mm in cross-section. Therefore, raw material is finely ground and separated from seeds and coarser matter, which would lower the extraction yield and purity of the final product. Thereafter, the product having a high water content is concentrated up to 15 Bx by means of common techniques such as centrifugation in a decanter.

In this case, the most interesting raw material is tomato and derivatives thereof. By way of a non-exhaustive example, the raw material to be used for the extraction of lycopene can be fresh tomatoes, certain high-lycopene tomatoes, and tomato processing waste, products and by-products. Tomato processing waste can comprise either the peels obtained from peeling of tomatoes intended for the production of tomato pulp cubes, or the peels resulting from the production of tomato sauce and paste. In all cases, tomato waste comprises tomato peels and seeds. The major content of lycopene is in the peels. The process can be applied either to the tomato waste also containing the seeds, or to the peels only following the separation from the seeds.

By way of a non-exhaustive example, tomato processing products can be selected from: non-peeled tomatoes, peeled tomatoes, tomato pulp cubes, tomato sauce, tomato juice, tomato paste.

According to a preferred method, the raw material is the waste obtained from peeling of tomatoes intended for the production of tomato pulp cubes. Such a waste comprises tomato peels rich in fibres and pulp attached thereto, as well as some seeds; this waste is high in lycopene (3.5 g/kg dried substance).

The use of waste from the industrial tomato processing as raw material for the present process allows to recover a material which is currently either used as an animal feed or even discarded; the extraction of lycopene from the waste of industrial tomato processing is an example of biorefinery, i.e. an innovative process which integrates with an established industrial process, in this case the industrial tomato processing, in order to recover substances with a high added value. Such achievement is an important progress in improving the sustainability of modern industry.

The plant material conditioned as described in the previous steps is then subjected to an enzymatic pre-treatment 30. The ratio between enzymatic formulation and raw material is in the range of from 0.08 to 0.17 ml/g raw material. The enzymatic formulation is diluted in water because the treatment is more effective when the plant material is evenly dispersed in water. A water/raw material ratio ranging from 1 to 2 ml/g, and preferably of 1.7 ml/g, is used to obtain a good dispersion of the raw material in water. The pre-treatment is carried out at a temperature in the range of from 40° to 70°C, and preferably of 50°C, with a pH usually ranging from 3 to 6, preferably of 4.5, in order to maximize the action of the enzymes. The enzymatic treatment will be continued for a time period ranging from 3 to 6 hours.

Once the aqueous phase 51 containing the enzymes has been extracted with the solvent, it is recovered and re-utilized for the subsequent cycles, since enzymes are not denatured by the solvent. The use of a small supplementation of fresh enzyme, for example of 5%, allows at least 12 recycles to be carried out. This means that, after 12 recycles, the real ratio between enzymatic formulation and raw material is <0.02 ml/g with a saving of 85%.

From an industrial point of view, the pre-treatment process occurs within closed stirring reactors with control of temperature at 50°C and in the absence of air to prevent lycopene oxidation. The recycling of enzymes is essential for the industrial process to be economically sustainable, and it ensures steady yields following to at least 12 recycles with the use of a small supplementation.

As for the choice of enzymes to be used, commercial products were used in various proportions and the following enzymatic formulation appeared to have the highest specific activity: 1/6 Peclyve PR or CP (pectinase activity), 1/6 Brewlyve 1500AXC (hemicellulase activity) and 4/6 Cellulyve 50 LC (cellulase activity). The use of an enzymatic mixture instead of each enzymatic formulation alone is extremely important because it has a synergic effect which is higher than the sum of the effects of each enzymatic formulation as used separately.

Once the pre-treatment step has been finished, solvent is directly added to the pre-treated mixture without any intervening separation, followed by the extraction step 40. Therefore, the extraction occurs in the presence of two non-miscible phases: the aqueous phase containing the enzymes, and the organic phase, i.e. the solvent, intended to extract the lycopene from the plant matrix. Adding the solvent directly to the pre-treated mixture without any intervening separation is critical both because this lowers the industrial costs, and because the presence of the aqueous phase during the extraction also improves the effectiveness of the extraction, since plant matrix is more evenly dispersed. The ratio between organic phase and aqueous phase is in the range of from 0.5 to 4 v/v, and preferably of 1.7 v/v. The ratio between solvent and plant raw material is in the range of from 1 to 5 ml/g, and preferably of 2.5 ml/g. The extraction temperature is in the range of from 25°C to 60°C, and preferably of 40°C. The duration of the extraction 40 is generally in the range of from 30 minutes to 4 hours, and preferably of one hour. The extraction can be repeated for several consecutive cycles, and it can be a batch or continuous process.

The separation 50 of the three phases - i.e. the organic phase 53, the aqueous phase 51 and the plant residue 52 - can be carried out by conventional separation techniques such as centrifugation, decanting, filtering, in order to separate the plant matrix, followed by the separation of the two non-miscible liquid phases. According to a preferred method, a three-phase centrifugal decanter known as Tricanter® is used to effectively separate the three phases in one step. The organic phase 53 is evaporated (60) under vacuum to recover the solvent and oleoresin. Oleoresin has a content of lycopene of about 4%. Once the aqueous phase 51 has been properly filtered, it is re-utilized for the enzymatic pre-treatment 30 of other raw material (recycling), while the solid residue 52 is sent to a de-solventization process and then used as an animal feed. The overall recovery yield for lycopene is in the range of from 75 to 90°/o.

The present process includes the purification of oleoresin to 4% lycopene by treatment with ethanol or acetone in a ratio ranging from 1:5 to 1:10 at room temperature, over a time period ranging from 10 to 60 minutes, followed by filtering and drying of crystals. The resulting crystals have a purity ranging from 40 to 90%, and the purification yield is about 90%.

The method according to the present invention will be better understood with reference to the following examples.

### Example 1:

100.0 kg of waste from industrial tomato processing with a lycopene content of 3.7 g/kg dry substance were subjected to the process of extraction of lycopene following an enzymatic pre-treatment.

The material was ground to obtain fragments smaller than 2 mm in size, then it was centrifuged to remove the serum excess. The centrifuged solid was subjected to an enzymatic pre-treatment with 150 litres of an enzymatic solution diluted to 10%, containing a mixture of enzymes comprising 1/6 Peclyve PR or CP (pectinase activity), 1/6 Brewlyve 1500AXC (hemicellulase activity), and 4/6 Cellulyve 50 LC (cellulase activity).

The enzymatic pre-treatment was carried out at a temperature of 50°C, pH 4.5, for 3 hours. Lycopene extraction was carried out by a subsequent addition of 255 litres of ethyl acetate at a temperature of 40°C over 1 hour.

The three phases - organic, aqueous and solid phases - were separated by centrifugation; organic phase was evaporated under vacuum, and the solvent was recovered for a subsequent re-utilization. 1,180 g of oleoresin were obtained, containing 47.2 g of lycopene at a concentration of about 4%.

The so-obtained oleoresin can be used either as an ingredient for the preparation of foods having functional characteristics, or as a dye.

### Example 2:

This example shows the effect of the composition of the enzymatic mixture on the extraction yield while leaving all the other process parameters unchanged for the steps of enzymatic pre-treatment and extraction with solvent.

Seven different enzymatic mixtures were used in order to identify the most effective composition in terms of lycopene extraction yield. The process parameters are the same as described in Example 1. The results are reported in Table 1 below.

**Table 1**

| Mixture | Peclyve PR | Ratio Cellulyve 50LC | Brewlyve 1500axe | % yield |
|---|---|---|---|---|
| 1 | 1 | | | 69 |
| 2 | | 1 | | 82 |
| 3 | | | 1 | 68 |
| 4 | 1/3 | 1/3 | 1/3 | 86 |
| 5 | 4/6 | 1/6 | 1/6 | 72 |
| **6** | **1/6** | **4/6** | **1/6** | **91** |
| 7 | 1/6 | 1/6 | 4/6 | 78 |

### Example 3:

The aqueous phase recovered by centrifugation of the three-phase mixture of Example 1 contains a solution of the enzymatic mixture used for the pre-treatment.

Such an aqueous phase was filtered in order to remove traces of suspended solids and then re-utilized for a subsequent cycle of pre-treatment and extraction.

The recycling of the aqueous phase containing the enzymatic mixture was repeated for 12 cycles without any substantial change in the extraction yield, using a supplementation of enzymatic mixture corresponding to 5% of the amount used in the first cycle in order to complement the matter and activity losses of enzymes.

The quantitative overall outcome of the re-utilization and supplementation is reported in Table 2, resulting in a decrease of 87% in the utilization of the amount of enzymatic formulation over 12 cycles.

**Table 2**

| Cycle | % extraction yield | Enzymatic formulation/raw material ratio |
|---|---|---|
| 1 | 95.8% | 0.150 |
| 2 | 88.0% | 0.079 |
| 3 | 94.9% | 0.055 |
| 4 | 93.6% | 0.043 |
| 5 | 95.4% | 0.036 |
| 6 | 97.2% | 0.031 |
| 7 | 95.9% | 0.028 |
| 8 | 90.9% | 0.025 |
| 9 | 91.7% | 0.023 |
| 10 | 94.2% | 0.022 |
| 11 | 91.1% | 0.020 |
| 12 | 89.7% | 0.019 |

### Example 4:

The oleoresin obtained according to the procedure of Example 1 was further treated in order to increase the titer of lycopene.

500 g of 4% lycopene oleoresin were treated with 5.0 litres of ethanol at room temperature for 10 minutes. Lycopene crystals were recovered by filtering and subsequent drying under vacuum. 26,4 g of 72% lycopene were obtained.

The crystals can be used to obtain various formulations, for example as an oil suspension or microencapsulated product.

## Claims

1. A method for extracting carotenoid substances from plant matrices, comprising the steps of: conditioning the plant matrix; treating the matrix with an enzymatic formulation in an aqueous phase; extracting the mixture comprising the matrix and the enzymatic formulation with an organic solvent; separating the aqueous phase from the organic phase and recovering the enzymatic formulation; removing the organic solvent and purifying the so-obtained carotenoid substances, wherein the enzymatic formulation includes pectinase, hemicellulase and cellulase enzymes having 1/6 pectinase activity, 1/6 hemicellulase activity and 4/6 cellulase activity.

2. The method according to claim 1, wherein the conditioning of the plant matrix is carried out by grinding and homogenization.

3. The method according to claim 2, wherein said conditioning is carried out on a refining machine with a sieve of 2 mm or less in size.

4. The method according to any one of claims 1 to 3, wherein said treatment with said enzymatic formulation is carried out at a temperature of from about 40°C to 70°C, and preferably of 50°C.

5. The method according to any one of the preceding claims 1 to 4, wherein said treatment with said enzymatic formulation is carried out at a pH of about 4.5.

6. The method according to any one of the preceding claims 1 to 5, wherein said enzymatic treatment is continued for a time period of from 3 to 5 hours.

7. The method according to any one of the preceding claims 1 to 6, wherein the solvent used in the extraction step is preferably a non-polar aprotic solvent.

8. The method according to claim 7, wherein said solvent is selected from the group consisting of hexane and ethyl acetate.

9. The method according to any one of the preceding claims 1 to 8, wherein said plant matrix comprises tomato waste from industrial tomato processing.

10. The method according to any one of the preceding claims 1 to 8, wherein said plant matrix comprises tomato processing products.

## Patentansprüche

1. Eine Methode zum Extrahieren von Carotinoiden aus pflanzlichen Matrices, die folgende Schritte umfasst: Konditionieren der pflanzlichen Matrix; Behandlung der Matrix mit einer Enzymformulierung in einer wässrigen Phase, Extrahieren der Mischung, einschließlich der Matrix und der Enzymformulierung mit einem organischen Lösungsmittel; Abtrennen der wässrigen Phase von der organischen Phase und Gewinnung der Enzymformulierung; Entfernen des organischen Lösungsmittels und Aufbereitung der so erhaltenen Carotinoide, wobei die Enzymformulierung Pektinase, Hemicellulase und Cellulase-Enzyme mit 1/6 Pektinase-Aktivität, 1/6 Hemicellulase-Aktivität und 4/6 Cellulase-Aktivität umfasst.

2. Die Methode gemäß Anspruch 1, wobei das Konditionieren der pflanzlichen Matrix durch Zerkleinerung und Homogenisierung durchgeführt wird.

3. Die Methode gemäß Anspruch 2, wobei besagtes Konditionieren auf einer Raffineriemaschine mit einem Sieb von 2 mm oder weniger groß durchgeführt wird.

4. Die Methode gemäß irgendeinem der vorherigen Ansprüche 1 bis 3, wobei besagte Behandlung mit besagter Enzymformulierung bei einer Temperatur von etwa 40°C bis 70°C und vorzugsweise bei 50°C durchgeführt wird.

5. Die Methode gemäß irgendeinem der vorherigen Ansprüche 1 bis 4, wobei besagte Behandlung mit besagter Enzymformulierung bei einem pH-Wert von etwa 4,5 durchgeführt wird.

6. Die Methode gemäß irgendeinem der vorherigen Ansprüche 1 bis 5, wobei besagte enzymatische Behandlung über einen Zeitraum von 3 bis 5 Stunden fortgeführt wird.

7. Die Methode gemäß irgendeinem der vorherigen Ansprüche 1 bis 6, wobei das bei den Schritten des Extrahierens verwendete Lösungsmittel vorzugsweise ein nicht-polarisches aprotisches Lösungsmittel ist.

8. Die Methode gemäß Anspruch 7, wobei besagtes Lösungsmittel von der Gruppe, die aus Hexan und Ethylacetat besteht, ausgewählt wird.

9. Die Methode gemäß irgendeinem der vorherigen Ansprüche 1 bis 8, wobei besagte pflanzliche Matrix Tomatenabfälle aus der industriellen Tomatenverarbeitung umfasst.

10. Die Methode gemäß irgendeinem der vorherigen Ansprüche 1 bis 8, wobei besagte pflanzliche Matrix Produkte der Tomatenverarbeitung umfasst.

## Revendications

1. Procédé pour extraire des substances caroténoïdes à partir de matrices végétales, comprenant les étapes consistant à: conditionner la matrice végétale; traiter la matrice avec une formulation enzymatique dans une phase aqueuse; extraire le mélange comprenant la matrice et la formulation enzymatique avec un solvant organique; séparer la phase aqueuse de la phase organique et récupérer la formulation enzymatique; éliminer le solvant organique et purifier les substances caroténoïdes ainsi obtenues, où la formulation enzymatique comprend du pectinases, de hémicellulases et des cellulases enzymes ayant 1/6 de activité de pectinase, 1/6 de activité de hémicellulase and 4/6 de activité de cellulase.

2. Procédé selon la revendication 1, où le conditionnement de la matrice de la plante est effectué par broyage et homogénéisation.

3. Procédé selon la revendication 2, où ledit conditionnement est effectué sur une machine de raffinage avec un tamis de 2 mm ou moins en taille.

4. Procédé selon l'une des revendications 1 à 3, où ledit traitement avec ladite formulation enzymatique est effectué à une température d'environ 40 °C à 70 °C, et de préférence de 50 °C.

5. Procédé selon l'une des revendications précédentes 1 à 4, où ledit traitement avec ladite formulation enzymatique est effectué à un pH d'environ 4,5.

6. Procédé selon l'une des revendications précédentes 1 à 5, où ledit traitement enzymatique est poursuivi pendant une période de temps allant de 3 à 5 heures.

7. Procédé selon l'une des revendications précédentes 1 à 6, où le solvant utilisé dans l'étape d'extraction est de préférence un solvant aprotique non polaire.

8. Procédé selon la revendication 7, où ledit solvant est choisi dans le groupe constitué d'hexane et d'acétate d'éthyle.

9. Procédé selon l'une des revendications précédentes 1 à 8, où ladite matrice végétale comprend des déchets de tomate issus de la transformation industrielle de la tomate.

10. Procédé selon l'une des revendications précédentes 1 à 8, où ladite matrice végétale comprend des produits issus de la transformation des tomates.
